# EUROPEAN PATENT SPECIFICATION

(11) **EP 1 904 001 B1**
(45) Date of publication and mention of the grant of the patent: **10.10.2012**
(21) Application number: 06750037.1
(22) Date of filing: 13.04.2006
(51) Int. Cl.: A61F 5/02

(54) **SPLINT OR SUPPORT WITH QUICK LOCATION TECHNIQUE**
SCHIENE ODER STÜTZE MIT SCHNELLER LOKALISIERUNGSTECHNIK
ATTELLE OU SUPPORT ET TECHNIQUE DE MISE EN PLACE RAPIDE

(30) Priority: 14.06.2005 US 153013
(43) Date of publication of application: 02.04.2008
(73) Proprietor: Ossur HF, 110 Reykjavik (IS)
(72) Inventor: GRIM, Tracy, E., Thousand Oaks, California 91362 (US); HENDERSON, Wendy, Ventura, California 93003 (US); IGLESIAS, Joseph, M., Newbury Park, California 91320 (US); CAMPOS, Michael, Sylmar, California 91342 (US); DOUBLEDAY, Walter, D., Jupiter, Florida 33488 (US); LONG, Kelly, M., Woodland Hills, California 91367 (US)
(74) Representative: Donné, Eddy
(86) International application number: PCT/US2006/013863
(87) International publication number: WO 2006/137962

(56) References cited:
- EP-A- 0 223 366
- US-A- 4 366 814
- US-A- 4 366 814
- US-A- 5 341 513
- US-A- 5 437 614

## Description

### BACKGROUND OF THE INVENTION

This invention relates to orthopaedic splints or supports.

In the field of hardenable orthopaedic splints and supports, the splints are normally rectangular in shape and are held on to the anatomy by a finishing tape of some kind wrapped along the entire length of the splint. To function properly, both the splint and finishing tape are soft and supple in order to conform to the contours of the anatomy. During application of the splint, it is necessary that the limb be held in a variety of positions. Frequently, lengthy splints must be used to properly immobilize the injured limb, and gravity causes one end or the other of the splint to fall away from the anatomy or merely shift away from the proper position. If one person is attempting to apply the splint, it is difficult to maintain the appropriate position for each particular injury during application. Usually it is necessary to ask for additional assistance to ensure proper application. More particularly, an extra set of hands is required to keep the splint in the desired location on the patient's anatomy until the outer securing or immobilization means has been applied.

From documents EP 0.223.366, which represents the closest prior art, and US 5.437.614 orthopedic support assemblies are already known with a support body that is at least partially impregnated with an activatable resin. For the patient's comfort, said support body can be made from a material with a certain extensibility. Said support assemblies are kept in place around a portion of the patient's anatomy by means of straps. These straps can be for instance of the VELCRO ® type.

### SUMMARY OF THE INVENTION

The present invention relates to a hardenable orthopedic assembly applied to a portion of a patient's anatomy, comprising a support body of at least one layer of material, the support body being at least partially impregnated with an activatable, hardenable resin; and a primary holding material attached to the support body; whereby the primary holding material is formed from a non-woven material and is removably attachable to a plurality of locations on the outer surface of the support body, the primary holding material having an initial unstretched length, and extending literally from at least one side of the support body, whereby the primary holding material at least partially wraps around the portion of the patient's anatomy over the outer surface of the support body, and whereby the primary holding material has a final length when stretched of about 100% which is at least about 30% longer than the initial lengths.

In accordance with the present invention a splint, blank or main support is provided with primary tacking arrangements to help hold the splint, blank, or main support in place, allowing the physician or technician the use of two free hands for quickly and easily adjusting the position of the splint, the blank or main support, if necessary without removing or loosening the primary tacking arrangements. The physician or technician then applies a secondary, more supportive securing arrangements to effectively hold the splint, blank or main support to the injured part of the patient's anatomy.

In accordance with one illustrative implementation of the invention, the primary tacking arrangements may include mating hook and loop type material such as VELCRO® type inserts or pads, or hook type patches used with a splint covering of unbroken loop (UBL) or other hook receivable material on the surface of the splint. In this regard the entire hook receivable surface acts as the loop portion of the hook and loop fastening arrangements.

In addition, with the splint or support normally being longitudinal in extent, laterally extending securing extensions may be provided with hook and loop, adhesive or other securing arrangements at the outer ends thereof. These lateral extensions may be formed as part of the hardenable portion of the splint, or as part of one layer thereof, or may be separate straps or the like, secured to the splint blank.

The secondary or "functional" securing arrangements may be in the form of a stretchable wrap such as an ACE® bandage, or may be straps, where the said arrangements are of sufficient strength to firmly hold the splint to the injured part of the anatomy during regular usage for extended periods of time.

Other features which may be included in implementation of the invention may involve the following:
1. The use of "spacer" type double knit material for the splint.
2. The use of an outer or secondary support such as an exo-skeleton type support, with associated straps serving as the functional securing arrangements.
3. The use of non-rectangular splints, with laterally extending areas forming the primary securing arrangements.
4. The use of a roll of splinting material, with lateral extensions spaced along its length.
5. The use of splint blanks with a thumbhole or another web space locater forming a part of the assembly.

In the field of splinting it is normally desirable to fully secure the splint or cast so that it will not come off or shift location during the normal course of daily activities. The primary holding or splint locating arrangements in the present invention are used when the splint blank is flexible and is being initially applied. These primary securing arrangements may be considered to be temporary "tacking" or locating arrangements, as they permit easy adjustment of the splint position, and are usually of insufficient strength to rigidly immobilize the splint for long term use by the patient.

Accordingly, when the term "tacking" is used in the present specification and claims, reference is being made to the primary holding arrangements which are of insufficient strength to fully secure the splint; and subsequent securing arrangements are normally required in addition to the "tacking." The "tacking" may be implemented, for example, by hook and loop fastening arrangements, by adhesive, by snaps, by hooks or any other suitable arrangement; and functional securing of the splint may be accomplished by lamination of the layers when the edges of hardenable material are overlapped, by wrapping with flexible tape, such as an ACE® bandage tape, by straps, or by any other securing arrangement of sufficient strength to properly immobilize the splint to the injured portion of the anatomy.

It is further noted that the use of a layer of UBL or hook-receivable material has the additional advantage of restricting the flow of the hardenable material, as set forth in, for example, U.S. Patent No. 6,824,522 (Henderson, et al.), titled "Hardenable Orthopaedic Supports".

In accordance with a method illustrating certain aspects of the invention, a splint or support is formed with sheet material impregnated with hardenable material, and with one or more primary laterally extending tacking extensions. The hardenable material is activated and the splint or support is held to the anatomy by engaging the laterally extending extensions; and the position and/or configuration of the impregnated sheet material is adjusted prior to the hardening of the hardenable sheet material while it is being loosely held by the tacking extensions; and the adjusted position is maintained while the hardening is accomplished.

Other objects, features and advantages will become apparent from a consideration of the following detailed description and the accompanying drawings.

### BRIEF DESCRIPTION OF THE DRAWINGS

Figure 1 is perspective view of a short arm splint embodiment illustrating the principles of the invention;
Fig. 2 is a partial cross-sectional view of part of the assembly of Fig. 1 prior to application;
Fig. 3 shows a splint roll with laterally extending tacking arrangements;
Fig. 4 shows a longitudinally extending splint or support blank with tacking arrangements extending from one side thereof;
Fig. 5 illustrates a splint blank with shaped tacking arrangements extending from both sides thereof;
Fig. 6 shows a non-rectangular splint or support blank with laterally extending tacking arrangements;
Fig. 7 is a showing of a splint blank with thumb holes within the laterally extending tacking arrangements;
Figs. 8 and 9 are perspective and cross-sectional views, respectively, of another embodiment of the invention;
Fig. 10 shows a splint or support, and an outer exo-skeletal construction;
Fig. 11 illustrates a splint or support for the forearm and thumb, with an attachment extending through the web space between the thumb and forefinger; and
Fig. 12 shows a splint for the upper and lower arm with an "anti-flexion" strap.

### DETAILED DESCRIPTION OF THE PREFERRED EMBODIMENTS

While the specification describes particular embodiments of the present invention, those of ordinary skill can devise variations of the present invention without departing from the inventive concept.

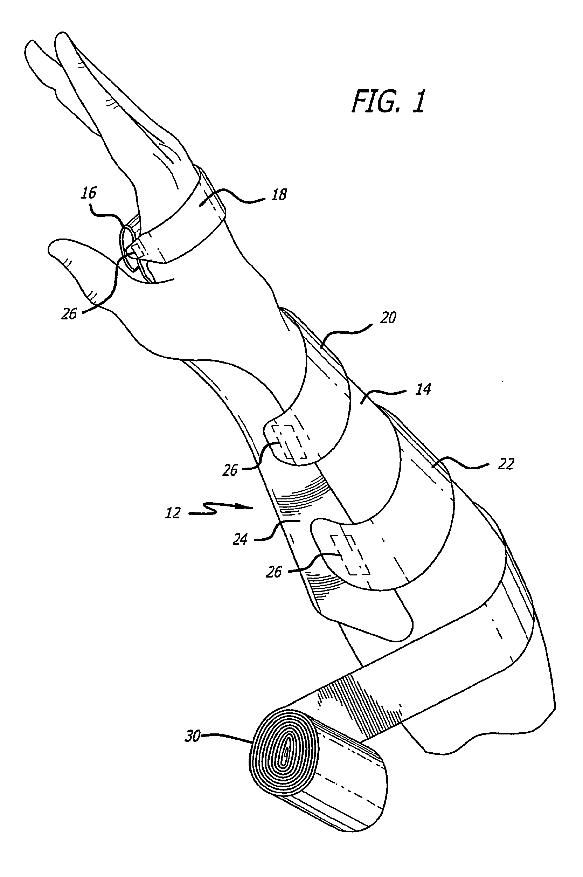

Referring more particularly to the drawings, Fig. 1 shows a splint or brace 12 mounted on the forearm 14 of a patient. The main part of the brace 12 is optionally folded over at reference numeral 16 at the palm of the patient's hand, and strap 18 extends over the back of the hand. The straps 18, 20 and 22 are secured to or are part of the main part 24 of the splint, and are provided with hook type fastener 26 on the underside of straps 18, 20 and 22 as shown in Fig. 1. The areas of the splint 12 underlying patches 26 are of hook receivable type material, so the straps 18, 20 and 22 are held or "tacked" in place as the splint 12 is applied. With the splint being held onto the forearm in an adjustable manner, the physician or medical technician can readily position the splint to the precise desired location, without the assistance of additional medical personnel.

The splint location is subsequently immobilized by wrapping the flexible, resilient tape 30 around the forearm and splint. This tape 30 may be of the type sold under the ACE® trademark.

In various embodiments, relative to the hook receivable type material underlying the hook type patches 26, it may either be in the form of hook receivable type patches secured to the splint 12 in selected areas; or alternatively, the entire splint may be covered with a layer of unbroken loop (UBL) or other hook receivable material, so that the hook type patches will engage and secure to the splint 24 at any convenient location.

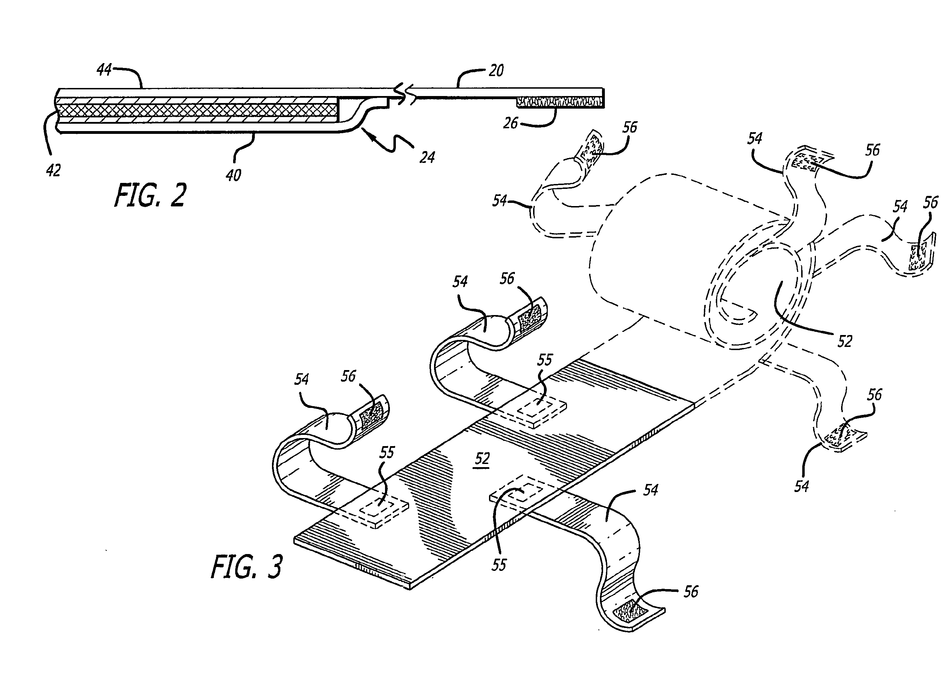

Furthermore, instead of VELCRO® type fasteners, adhesive, snaps, or hooks, for example, could be employed for tacking the straps 18, 20 and 22 in place. Figure 2 is a schematic cross-sectional view of a part of the assembly of Fig. 1 prior to application. In Fig. 2, the main portion 24 of the splint 12 includes an inner padding layer 40, and a central layer of hardenable material 42 preferably made of a double knit spacer material. The double knit spacer material includes upper and lower woven or knit layers, with an integral matrix of fibers or filaments interconnecting the upper and lower layers. Such double knit materials are disclosed in, for example, U.S. Patent No. 6,139,513 (Grim, et al.). An additional layer of unbroken loop (UBL) or hook receivable material 44 is adjacent to the upper surface of the spacer material 42. The strap 20 may be formed of separate material, or may be a continuation of any one of the layers of the main body 24 of the splint blank. It may also be either permanently attached or removably attached, using VELCRO®, hooks, etc. At the outer end of strap 20 is a patch 26 of hook type material for securing to underlying hook receivable type material 44.

The hardenable layer 42 is preferably impregnated with a water activatable material such as urethane. The layer of hook receivable or UBL material 44 may inhibit the transfer of the water activatable material to the outer surface of the splint.

Figure 3 shows a continuous roll of a splinting material 52 with laterally outwardly extending tacking arrangements 54 having patches 56 of hook type material located at the ends thereof. The main body of the splint roll 52 consists of at least one layer of material impregnated with a water activatable material. The outer surface of the splint roll 52 may be covered with hook receivable material or may be provided with patches of hook receivable type material for tacking engagement with the hook type patches 56. The tacking strips 54 may be in the form of separate strips with hook type patches 55 at the inner ends of strips 54 as well as on the outer ends of strips 54 where hook type patches 56 are provided. This gives flexibility in the location of the tacking arrangements of the splint onto the anatomy. Alternatively, the tacking strips 54 may be permanently secured to the splinting material 52 or the strips 54 may be an extension or continuation of one of the layers of splinting material 52. A splint or support may be cut from the roll at any desired length to form a blank of the desired size.

The preferred splint roll configuration is an inner activatable material of at least one layer with an outer covering material on one side and a padding layer on the other. The outer covering is preferably made from a hook receivable material, such as a UBL material, and the padding may consist of at least one layer of either a double knit spacer material, foam, or a nonwoven material. Throughout the length of the splint are tacking arrangements spaced at equal intervals. The extensions may have hook type arrangements on both ends so that they would be removable and movable with respect to the splint assembly. It is also preferable that the tacking arrangements be made of a stretchable material and have a configuration that would ensure proper securing yet not cause any discomfort to the end user. An acceptable material to use would be a nonwoven because of its cost effectiveness and low profile. The splint can be cut to usable lengths prior to packaging or may be provided in long lengths and may be cut to the appropriate size immediately prior to application.

In the case of all of the embodiments as disclosed in this specification, the construction and alternatives as disclosed herein may be used in each of the constructions intended for specific application. Thus, for example, after cutting off a length of the splint roll 52, activating it, and tacking it in place, the splint may be wrapped with a flexible wrap, to functionally secure the splint.

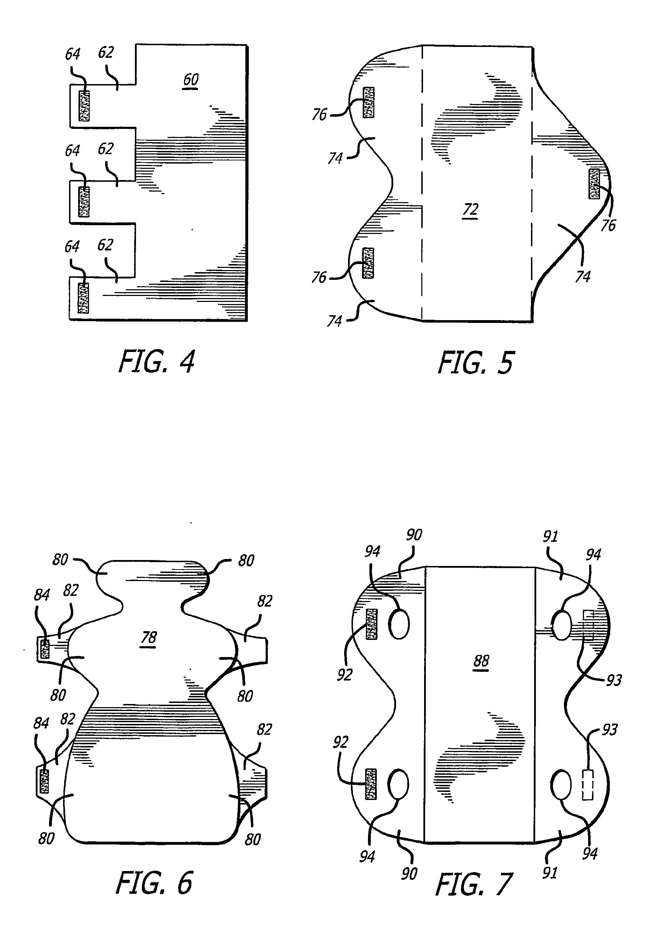

Figures 4 through 7 show further embodiments of the invention with different geometric configurations. In Fig. 4 the longitudinally extending main blank 60 has laterally extending tacking arrangements 62 extending from one side. Hook type patches 64 are provided at the outer ends of the tacking extensions 62, to mate with underlying hook receivable type material. In use, these extensions 62 are wrapped around the anatomy and secured to either the outer hook receivable covering of the splint 60 or to strategic areas where hook receivable patches have been placed on the surface of the blank 60. The lateral extensions may be an extension of the covering material, padding material, hardenable material, or may be made from an entirely separate material attached to the splinting assembly. The extensions may also be either permanently attached to the splint, i.e., by sewing, or may be removably attached using arrangements such as a hook type fastener.

In Fig. 5 the main longitudinally extending splint blank 72 has shaped laterally extending tacking arrangements 74 with hook type patches 76. In this particular example, the shaped tacking arrangements are staggered and sufficiently wide to allow for complete coverage of the arm when in use, giving circumferential compression and a broader distribution of the forces exerted by the arrangements than would be provided by a narrower strip.

In Fig. 6 the hardenable portion of the splint blank 78 has a "snowman" like configuration, with rounded lateral zones 80. The rounded or contoured shape of the hardenable blank can be strategically designed to better fit and mold to the complex contours of the anatomy than a rectangular splint with no contours. To these lateral zones 80 are secured laterally outwardly extending tacking extensions 82 with mating hook patches 84 and hook receivable patches (or a UBL layer) underlying these extensions when the splint is applied. Instead of having one set of extensions as in Fig. 5, Fig. 6 shows two sets of extensions which will both wrap partially around the anatomy and mate with each other to provide the tacking arrangements.

The splint blank of Fig. 7 has a longitudinally extending central zone 88 and shaped laterally extending zones 90 and 91. Having shaped extensions instead of rectangular strips provides for wider coverage and more pressure distribution over the strapping area, thus reducing potential pressure points associated with a thinner strip. The laterally extending zones 90 have hook type patches 92 to engage mating hook receivable type material 93 on the paired zones 91 on the other side of the blank 88. Alternatively, the entire surface of zones 90 and 91 may be of a hook receivable material and thus the patches 93 would be unnecessary. In use, the thumb is slid through one of the pre-made thumb holes 94 which provide assistance in locating the blank 88 on the forearm of the patient. Multiple thumb holes are provided so that application is possible, regardless of the initial orientation of the splint when removed from the package. Other methods of creating a thumb hole include providing slits in the zones 90 and 91 or making the laterally extending zones out of a tearable material which can be torn to receive a thumb in the appropriate location immediately prior to application.

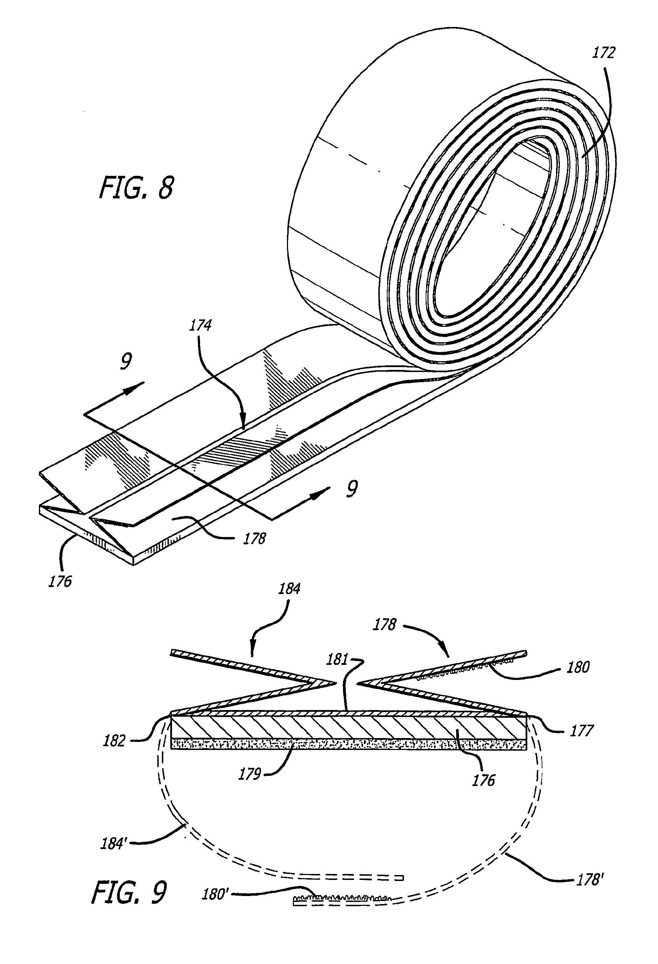

Referring now to Figs. 8 and 9 of the drawings, Fig. 8 is a perspective view of a water hardenable splint or support in roll form; and Fig. 9 is a cross-sectional view taken along the plane indicated at 9 - 9 in Fig. 8. In Fig. 8, the roll 172 is partially unrolled to show a section 174 which may be cut off at the desired length.

As shown to advantage in Fig. 9 the splint or support may have a base 176 of spacer material impregnated with water hardenable material. Extending along and secured to one edge 177 of the base 176 is a strip of folded sheet material 178 which has hook material 180 located on one surface thereof as indicated in Fig. 9. From the other edge 182 of the base 176, a second folded strip 184 is secured. The strip 184 may be formed of hook receivable sheet material, such as UBL material or a non-woven fabric.

Shown in dashed lines are the strips 178' and 184' in an unfolded configuration, to extend around a portion of the anatomy such as a forearm or lower leg, for example. The hook material 180' engages the lower surface of the unfolded hook receivable strip 184' to hold the base member 176 onto the desired part of the anatomy. This arrangement permits tacking of the base strip 176 in place while the physician or medical technician is adjusting the position of the splint or support. As shown in Fig. 9, the tacking arrangements extend the entire length of the splint. It is possible to cut these arrangements into any configuration that works best for the user. For example, the elongated arrangements can be slit at various locations to form more narrow straps which can be wrapped around the anatomy. It is also possible to remove any section that is unneeded by cutting or tearing.

As shown in Fig. 9, padding material 179 may be provided underlying the strip 176 which is impregnated with hardenable material. With this arrangement, the strip of hardenable material 176 does not directly engage the patient's skin. Incidentally, instead of being formed of spacer type double knit material, the strip 176 may be formed of multiple layers of single knit material. The padding material 179 may be composed of a layer of double knit material. The strip 176 may be covered on the other side with a layer of hook receivable material 181 which may have extensions such as those shown at 178 and 184. Thus, the material 181 may be integral with strips 178 and 184; or the strips 184 and 178 may be separately secured to the edges of strip 176. Alternatively, the padding material may extend outwardly to form the securing strips 178 and 184. The foregoing are merely examples of alternative embodiments and many different combinations are possible which incorporate the various configurations discussed above.

The assembly as shown in Figs. 8 or 9, may subsequently be wrapped with an ACE® type strip material.

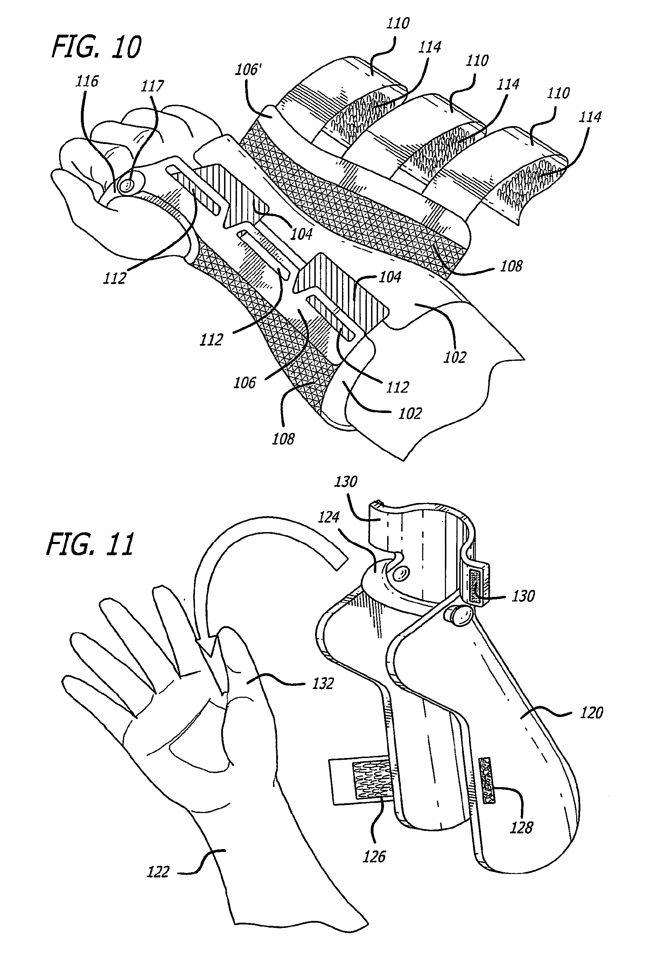

The embodiment of Fig. 10 includes an inner blank 102 with tacking arrangements 104 which engage the underlying opposing edge of inner blank 102. A pre-shaped outer external wrap can serve as the functional securing arrangements. For example, Fig. 10 is one specific example of these arrangements where exo-skeletal members 106 and 106' are formed of fairly stiff plastic and are molded to the flexible material 108 which extends around and over the inner splint 102. The plastic strip 106' is molded to the flexible material 108 at the edge of material 108 away from exo-skeletal member 106. During application, the functional securing straps 110 extend through loops 112 and are then folded back upon themselves and secured in place by mating hook and loop securing surfaces 114 on the outer ends of straps 110. The outer exo-skeletal member is further immobilized by the attachment 116 extending through the web space between the thumb and forefinger. The attachment 116 may be secured to member 106 by the rivet 117 and an equivalent rivet (not shown) on the opposite side, or may be adjustably secured using hook and loop type fasteners.

Figure 11 shows a water hardenable splint blank 120 for providing splinting for the wrist and forearm 122. The blank 120 is provided with an attachment 124 for extending through the web space between the thumb and the forefinger. Preconnecting the attachment 124 to span the web allows for much easier and intuitive application of the support. The attachment or strap 124 may be riveted or sewn, for example, to splint blank 120. The attachment 124 can be made from a thermoplastic, fabric, fiberglass, or combination of these materials, for example. It is also possible for the attachment to be made from a material which has been treated to be water activatable, or alternatively may be inactivatable and remain soft and flexible for increased comfort during wear. This attachment may be permanently or removably attached and may have adjustment means, for example, hook and loop type fasteners, to accommodate different sizes of anatomy. In addition, the tacking strap 126 having a patch of hook type material thereon, engages the hook receivable type patch 128 to quickly hold the blank 120 in place. The flaps 130 are wrapped around the thumb 132 to complete the splinting of the forearm and thumb, and may incorporate hook and loop type fasteners for quick securing during application. Functional securing in this select area is then achieved by the lamination of the layers of overlapping activatable flaps 130.

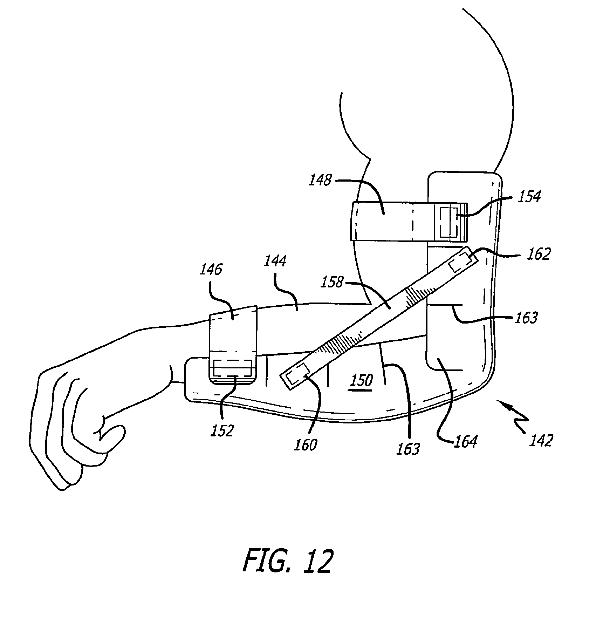

Figure 12 shows a further embodiment illustrating the principles of the invention. In this embodiment, the splint 142 is mounted on the patient's arm 144. The tacking arrangements 146 and 148 are secured to the splint 150 at the rear of the splint. These securing points are not visible in Fig. 12 as they are on the opposite side of the arm. The tacking arrangements 146 and 148 have hook type patches 152 and 154 mounted on the ends thereof, which mate with a complementary hook receivable type material on the splint. This loop type material may be in the form of a patch on the splint 142, or the entire body 150 of splint 142 may have an outer layer of UBL or other hook receivable material thereon with which the hook type patches 152 and 154 engage and mate.

With the outer surface being covered with a UBL or other hook receivable material, an anti-flexing strap 158 may be utilized. The anti-flexing strap 158 may have hook type patches 160 and 162 at its ends, to mate with hook receivable type material on the body 150 of the splint. During application, the anti-flexing strap 158 aids in securing the arm and limiting the ability of the arm and splint to flex until it has hardened. After hardening, it will help reinforce the splint and keep the elbow at a ninety degree angle. If desired, the anti-flexion strap 158 may be permanently secured to splint 150 at one end thereof. As shown, the anti-flexion strap is used to help stabilize an elbow. However, it may also be used to stabilize other necessary portions of the anatomy such as the ankle.

An additional feature shown in Fig. 12 is the ability to create slits 163 on both sides of the entire length of the splint. The slits allow for the splint to contour around complex curves of the anatomy without bunching and wrinkling. As shown in Fig. 12 when the splint is folded around the elbow, the splint material adjacent the slits overlap in the area 164 forming a lower profile splint with fewer wrinkles which might otherwise cause the user discomfort.

After the splint 142 is properly mounted in place, the entire splint assembly may be wrapped with a secondary, flexible, resilient tape such as an ACE® type wrap, to functionally secure the splinting arrangements.

In regard to the materials used for fabricating the carious layers of the above mentioned splinting assemblies, the primary tacking arrangements are preferably made from a nonwoven material. Nonwoven materials are typically very inexpensive and may be made to be hydrophobic which is very advantageous. The tacking materials may also be made of a knitted material, woven fabric, or foam. It is also beneficial if the primary tacking arrangements are low profile, stretchable and tearable. The activatable layer can be composed of a double knit spacer material, multiple layers of a single knit material foam laminate, nonwoven material, woven material, or any suitable material that will achieve sufficient strength upon hardening.

As seen in the exemplary embodiments of Figs. 1-3, there is a main support body 24 that is at least partially impregnated with an activatable hardenable resin. The process of applying the main support body or splint 24 or 52 to a portion of a patient's anatomy such as the forearm in Fig. 1 is simplified by the use of the primary holding or tacking material 20 or 54. In various preferred embodiments, the primary holding or tacking material is made from a thin material of a thickness ranging from about 0.13-0.76 mm 0.005-0.03 inch and more preferably about 0.25 mm (0.01 inch) thick to ensure a low profile and mimimal bulk.

The primary holding material should preferably be made from a material that is porous to ambient air so that it is breathable and thus comfortable to the patient. To achieve a good balance of air porosity versus hold strength and fabric integrity, the density of the primary holding material preferably falls within a range of 25 - 110 g/m² and more preferably 50 - 65 g/m², including all values at and within the specified outer limits.

Further, the primary holding material may be treated to be hydrophobic, and as according to the invention, the primary holding material is formed from a nonwoven material to keep down expense. Specifically, the nonwoven materials are typically made from polyester, rayon, or polypropylene fabrics. Alternatively, sheets or strips of natural rubber, latex, thermoplastic rubber (TPE) such as SANTOPRENE, HYDREL, KRAYTON, or the like may be used. Most of these materials are inherently hydrophobic. In addition, the nonwoven materials can optionally be treated, for example, with Scotchguard or a Teflon coating to enhance hydrophobic properties.

The primary holding material is preferably not made entirely of the aforementioned materials, but rather such materials are incorporated into the nonwoven fabric to give it stretch properties. Most stretchable nonwoven materials are not just one layer of fabric but some type of laminated structure to allow for stretchability. It is therefore preferable that the primary holding material be a laminate of stretchable nonwoven materials selected from one or any combination of the foregoing listed materials. The nonwoven material can either be stretchable throughout its entire length or only have certain sections that are stretchable and other sections that are not stretchable. A region of the primary holding material may further be stretchable in one direction yet non-stretchable in another direction, such as longitudinally versus transversely. The non-stretchable sections or regions help keep the primary holding material or strap from "necking down" when it is stretched and wrapped around the patient's anatomy. The "necking down" of the primary holding material leads to the unwanted turniquette effect.

Exemplary width dimensions for the primary holding material ranges from about 2.5 cm to 15 cm (1 inch to 6 inches) wide, including all sizes therebetween. To enable tacking the primary support material to the splint support body, brace, or blank, the former may be made from hook receivable material, UBL, or napped fabric, or include patches of the hook or loop portion from a hook and loop type fastener such as VELCRO®.

Alternatively, a means for tacking may be used to attach the primary holding material to the splint support body, brace, or blank. For example, the means for tacking 26 shown in Figs. 1-2, and items 55, 56 in Fig. 3, may be a complementary hook and loop fastener such as VELCRO®, or may be a hook receivable cover 24 seen in Fig. 2, or could be a weak adhesive such as that used in COBAN. A hook patch 26 on the primary holding material 20 may be used to attach the primary holding material 20 to a hook receivable or UBL cover 44 as seen in Fig. 2. In various embodiments, the cover 44 shown in Fig. 2 can be a UBL or hook receivable material on the outside of the entire splint support body, brace, or blank 24, 52, thus allowing the primary holding material or strap 20 to be secured anywhere thereto. Or the cover 44 may have patches of UBL or hook receivable material on the outside of the splint support body 42, brace 24, or blank 60, for example. The means for tacking may itself be welded, bonded, sewn, riveted, or mechanically joined to the fabric or substrate forming the primary holding material, or it may be formed as part of the substrate or primary holding material fabric.

Preferably, the primary holding material is stretchable, where its length under a tensile load L may increase up to about 50 % or greater of its unstretched, initial length. This amount of stretch may be measured on the basis of the entire structure or only a portion thereof. Conventional stretchable materials used with hook and loop type fasteners can only stretch up to about 40 % of its initial length. The stretching is measured from the percent of elastic increase from its unstretched, initial length, where the stretched length is achieved just prior to any plastic or permanent deformation, or failure in the material in the form of tearing.

In addition, the primary holding material exhibits limited rebound, i.e., resistance to stretch. This feature prevents a turniquette effect on the wearer patient, which would otherwise impede circulation or movement of the limb, joint, or body part. Therefore, it is preferable that the primary holding material can be stretched under tensile load L to an increase in length of about 5 cm (2 inches) or 30 % of its initial unstretched length under a tensile force of about 1.5 lbf. (6.7 N); in alternative embodiments, that tensile load L may be about 1.3 lbf. (6 N) down to 1.0 lbf. (4.5 N), or even less to achieve a 30 % stretch. When the primary holding material is tested by stretching it 100 % and held for about 5 minutes, the final length is about 30 % or greater than the initial, unstretched length.

Furthermore, the primary holding material is designed to tear before causing the turniquette effect. To achieve this behavior, the primary holding material has an ultimate tensile strength of about 10 lbf. (44.5 N) or less (but greater than 0 lbf., of course), and more preferably of about 3 lbf. (13.4 N), including all values therebetween, when tearing is initiated. The primary holding material can resist complete detachment or separation into two discrete pieces from the tensile loads L that are less than about 10 lbf. (44 N) and more preferably about 3 lbf. (13.4 N). In addition, the tearing when the ultimate tensile strength is exceeded may be designed to occur anywhere in the primary holding material, or could be purposely designed to propagate at precise tear regions. These tear regions may even have optional perforations or have precuts at the edge, for example. Empirical observations have found that the foregoing stretch and tear characteristics are effective for minimizing and virtually eliminating the unwanted turniquette effect.

It is to be understood that the foregoing detailed description and the embodiments shown in the drawings are illustrative embodiments of the invention. Various alternatives and modifications may be made without departing from the scope of the invention. Thus, by way of example and not of limitation, in most of the embodiments shown herein, and specifically the embodiments of Figs. 1 - 7 and 11, the secondary, functional securing may be accomplished using flexible elastic tape 30 as indicated in Fig. 1, or straps 110 as suggested in Fig. 10. Further, immobilization straps may be employed in all disclosed embodiments, in addition to the tacking arrangements as shown. Regarding the construction of the blanks, they may be of other forms than that shown in Fig. 2, and may include multiple layers of high strength material, for example, instead of a spacer material. Also, concerning hook receivable material, it may be in the form of patches of VELCRO® material, UBL material, or napped material, for example. More generally the variations suggested by any of the drawings or related description are applicable to the other embodiments disclosed herein. Instead of being water hardenable, the splints may include two materials with arrangements for combining the materials to initiate hardening. Accordingly, the present specification is not limiting the invention precisely as described in detail hereinabove and shown in the drawings.

While the specification describes particular embodiments of the present invention, those of ordinary skill can devise variations of the present invention without departing from the scope of the invention.

## Claims

1. A hardenable splint assembly (12) comprising: a support body (24, 52) of at least one layer of material, the support body being at least partially impregnated with an activatable, hardenable resin and at least one tacking arrangement (20, 22, 54) for temporarily holding the assembly on a portion of the patient's anatomy before and during securing it by any securing arrangement to this portion of the patient's anatomy, in which the tacking arrangement (20, 22, 54) is at one end attached to the support body (24), **characterized in that** the tacking arrangement (20, 22, 54) is formed from a non-woven material and is removably attachable with its other end to a plurality of locations on the outer surface of the support body (24), whereby the tacking arrangement (20, 22, 54) has an initial unstretched length, and extends laterally from at least one side of the support body (24), and whereby the tacking arrangement (20, 22, 54) at least partially wraps around the portion of the patient's anatomy over the outer surface of the support body (24) and whereby the tacking arrangement (20, 22, 54) has a final length when stretched about 100% which is at least about 30% longer than the initial length.

2. The hardenable orthopedic assembly according to claim 1, **characterized in that** the support body (24) is at least partially covered by one of a padding and a covering formed from an unbroken loop material on an outer side thereof.

3. The hardenable orthopedic assembly according to claim 1, **characterized in that** the tacking arrangement (20, 22, 54) is breathable and porous to ambient air.

4. The hardenable orthopedic assembly according to claim 1, **characterized in that** the tacking arrangement (20, 22, 54) is substantially hydrophobic.

5. The hardenable orthopedic assembly according to claim 1, **characterized in that** the tacking arrangement (20, 22, 54) can be stretched up to about 30% of the initial length with a tensile load L of about 4,5 N to about 6,7 N.

6. The hardenable orthopedic assembly according to claim 1, **characterized in that** the support body (24) is sandwiched in between a covering and padding.

7. The hardenable orthopedic assembly according to claim 1, **characterized in that** the support body (24) includes a hook receivable material.

8. The hardenable orthopedic assembly according to claim 1, **characterized in that** the tacking arrangement (20, 22, 54) has a width of about 2,54 cm to about 15,24 cm.

9. The hardenable orthopedic assembly according to claim 1, **characterized in that** the tacking arrangement (20, 22, 54) can be stretched up to about 30% of the initial length with a tensile load L of about 4,5 N.

10. The hardenable orthopedic assembly according to claim 1, **characterized in that** the support body (24) includes a strip form and can be arranged into a roll.

## Patentansprüche

1. Härtbare Schienenanordnung (12), umfassend: einen Stützkörper (24, 52) aus mindestens einer Materiallage, wobei der Stützkörper mindestens teilweise mit einem aktivierbaren, härtbaren Harz imprägniert ist, und mindestens eine Anheftanordnung (20, 22, 54) zum zeitweiligen Festhalten der Anordnung an einem Teil der Anatomie des Patienten, bevor und während sie mittels gleich welcher Sicherungsanordnung an diesem Teil der Anatomie des Patienten gesichert wird, wobei die Anheftanordnung (20, 22, 54) an einem Ende an dem Stützkörper (24) befestigt ist, **dadurch gekennzeichnet, dass** die Anheftanordnung (20, 22, 54) aus einem Textilverbundstoff-Material ausgebildet ist und mit ihrem anderen Ende lösbar an einer Vielzahl von Stellen an der Außenseite des Stützkörpers (24) befestigt werden kann, wobei die Anheftanordnung (20, 22, 54) eine ursprüngliche ungedehnte Länge aufweist und sich lateral von mindestens einer Seite des Stützkörpers (24) erstreckt, und wobei die Anheftanordnung (20, 22, 54) mindestens teilweise um den Teil der Anatomie des Patienten über die Außenseite des Stützkörpers (24) herumgeschlagen wird und wobei die Anheftanordnung (20, 22, 54) eine letztendliche Länge, wenn sie um etwa 100% gedehnt ist, aufweist, die mindestens etwa 30% länger als die ursprüngliche Länge ist.

2. Härtbare orthopädische Anordnung nach Anspruch 1, **dadurch gekennzeichnet, dass** der Stützkörper (24) mindestens teilweise durch eines einer Polsterung und einer Abdeckung, gebildet aus einem Material mit ununterbrochenen Schlaufen, an einer Außenseite davon bedeckt ist.

3. Härtbare orthopädische Anordnung nach Anspruch 1, **dadurch gekennzeichnet, dass** die Anheftanordnung (20, 22, 54) atmungsfähig und porös für Umgebungsluft ist.

4. Härtbare orthopädische Anordnung nach Anspruch 1, **dadurch gekennzeichnet, dass** die Anheftanordnung (20,22,54) im Wesentlichen hydrophob ist.

5. Härtbare orthopädische Anordnung nach Anspruch 1, **dadurch gekennzeichnet, dass** die Anheftanordnung (20, 22, 54) mit einer Zugbelastung L von etwa 4,5 N bis etwa 6,7 N bis auf etwa 30% der ursprünglichen Länge gedehnt werden kann.

6. Härtbare orthopädische Anordnung nach Anspruch 1, **dadurch gekennzeichnet, dass** der Stützkörper (24) sich sandwichartig zwischen einer Abdeckung und Polsterung befindet.

7. Härtbare orthopädische Anordnung nach Anspruch 1, **dadurch gekennzeichnet, dass** der Stützkörper (24) ein Häkchenaufnahmematerial beinhaltet.

8. Härtbare orthopädische Anordnung nach Anspruch 1, **dadurch gekennzeichnet, dass** die Anheftanordnung (20, 22, 54) eine Breite von etwa 2,54 cm bis etwa 15,24 cm aufweist.

9. Härtbare orthopädische Anordnung nach Anspruch 1, **dadurch gekennzeichnet, dass** die Anheftanordnung (20, 22, 54) mit einer Zugbelastung L von etwa 4,5 N bis auf etwa 30% der ursprünglichen Länge gedehnt werden kann.

10. Härtbare orthopädische Anordnung nach Anspruch 1, **dadurch gekennzeichnet, dass** der Stützkörper (24) eine Streifenform beinhaltet und zu einer Rolle angeordnet werden kann.

## Revendications

1. Assemblage d'attelle durcissable (12) comprenant :
un corps de support (24, 52) constitué par au moins une couche de tissu, le corps de support étant imprégné au moins en partie avec une résine durcissable qui peut être activée, et par au moins un arrangement d'attache (20, 22, 54) pour le maintien temporaire de l'assemblage sur une portion de l'anatomie du patient avant et pendant sa fixation via un quelconque arrangement de fixation à cette portion de l'anatomie du patient, l'arrangement d'attache (20, 22, 54) étant fixé, à une extrémité, au corps de support (24), **caractérisé en ce que** l'arrangement d'attache (20, 22, 54) est réalisé à partir d'un non-tissé et peut être fixé de manière amovible avec son autre extrémité à plusieurs endroits sur la surface externe du corps de support (24), l'arrangement d'attache (20, 22, 54) possédant une longueur initiale à l'état non étiré et s'étendant en direction latérale à partir d'au moins un côté du corps de support (24), et par lequel l'arrangement d'attache (20, 22, 54) entoure au moins en partie la portion de l'anatomie du patient par-dessus la surface externe du corps de support (24) et par lequel l'arrangement d'attache (20, 22, 54) possède une longueur finale, à l'état étiré à concurrence d'environ 100 %, qui est supérieure à la longueur initiale à concurrence d'au moins environ 30 %.

2. Assemblage orthopédique durcissable selon la revendication 1, **caractérisé en ce que** le corps de support (24) est recouvert au moins en partie d'un rembourrage et d'un recouvrement formé à partir d'un tissu bouclette ininterrompu, sur son côté externe.

3. Assemblage orthopédique durcissable selon la revendication 1, **caractérisé en ce que** l'arrangement d'attache (20, 22, 54) est de type respirant et perméable à l'air ambiant.

4. Assemblage orthopédique durcissable selon la revendication 1, **caractérisé en ce que** l'arrangement d'attache (20, 22, 54) est essentiellement hydrophobe.

5. Assemblage orthopédique durcissable selon la revendication 1, **caractérisé en ce que** l'arrangement d'attache (20, 22, 54) peut être étiré jusqu'à environ 30 % de sa longueur initiale avec une charge de tension L d'environ 4,5 N à environ 6,7 N.

6. Assemblage orthopédique durcissable selon la revendication 1, **caractérisé en ce que** le corps de support (24) est intercalé entre un recouvrement et un rembourrage.

7. Assemblage orthopédique durcissable selon la revendication 1, **caractérisé en ce que** le corps de support (24) englobe une matière de réception à crochets.

8. Assemblage orthopédique durcissable selon la revendication 1, **caractérisé en ce que** l'arrangement d'attache (20, 22, 54) possède une largeur d'environ 2,54 cm à environ 15,24 cm.

9. Assemblage orthopédique durcissable selon la revendication 1, **caractérisé en ce que** l'arrangement d'attache (20, 22, 54) peut être étiré jusqu'à environ 30 % de sa longueur initiale avec une charge de tension L d'environ 4,5 N.

10. Assemblage orthopédique durcissable selon la revendication 1, **caractérisé en ce que** le corps de support (24) englobe une forme de bande et peut être arrangé pour obtenir un rouleau.
